# FASCICULE DE BREVET EUROPEEN

(11) **EP 0 487 429 B1**
(45) Date de publication et mention de la délivrance du brevet: **27.03.1996**
(21) Numéro de dépôt: 91403161.2
(22) Date de dépôt: 22.11.1991
(51) Int. Cl.: A61N 1/08, A61N 1/365, A61N 1/39

(54) **Dispositif de prévention des défaillances cardiaques**
Einrichtung zur Verhinderung von Herzfunktionstörungen
Device for preventing cardiac malfunctions

(30) Priorité: 23.11.1990 FR 9014643
(43) Date de publication de la demande: 27.05.1992
(73) Titulaire: Zacouto, Fred, F-75015 Paris (FR)
(72) Inventeur: Zacouto, Fred, F-75015 Paris (FR)
(74) Mandataire: Bernasconi, Jean

(56) Documents cités:
- EP-A- 0 348 271
- EP-A- 0 387 363
- EP-A- 0 398 548
- US-A- 4 473 078

## Description

L'invention a trait à un dispositif de prévention d'une part, des troubles du rythme cardiaque, notamment tachycardies ou tachyarythmies ventriculaires, et fibrillations ventriculaires, cause de mort subite, et d'autre part insuffisances cardiovasculaires mécaniques, thrombose et hémorragies.

Le traitement automatique des troubles cardiaques à l'aide de dispositifs implantés présente essentiellement un aspect curatif ou palliatif momentané, sans prévenir les rechutes ou soigner la cause. Les premiers stimulateurs cardiaques ont été conçus pour suppléer aux insuffisances de l'électrogenèse cardiaque, notamment en cas de bradycardie par bloc auriculo-ventriculaire. Le traitement automatique des tachycardies ou des tachyarythmies, notamment ventriculaires, s'est avéré difficile et il a fallu attendre l'invention du stimulateur orthorythmique (brevet US-A-3.857.399 et US-A-4.052.991) par le déposant, pour parvenir à réduire automatiquement, avec de grandes chances de succès, les tachycardies ou tachyarythmies. L'action curative de ce dispositif, qui intervient en cas de détection d'une tachycardie ou tachyarythmie naissante, présente aussi, dans une certaine mesure, un aspect préventif, car elle empêche, souvent, une tachycardie détectée de durer suffisamment pour devenir dangereuse. Néanmoins, il reste souhaitable de pouvoir prévenir toute naissance de tachycardie. C'est pour cette raison que ces dernières années ont vu le développement d'un grand nombre de défibrillateurs basés sur la défibrillation automatique inventée par le déposant (FR-A-1237702 du 11.07.1953) et destinés à détecter sélectivement la survenue d'une fibrillation ventriculaire et à générer un choc électrique de défibrillation.

Le déposant a déjà cherché à mettre au point des dispositifs de prévention des accidents cardiaques. L'un de ces dispositifs décrit dans le brevet US-A-4.552.150 (1983), teste périodiquement l'excitabilité du myocarde par l'envoi d'impulsions infraliminaires, normalement suivies d'aucune réponse, afin de procéder à une régulation du myocarde par stimulation électrique lorsque le seuil d'excitabilité baisse trop.

Un autre dispositif conçu par le déposant dans la demande de brevet EP-A-O.348 271 (Paris 1988) permet de détecter les risques de thrombose ou d'ischémie immédiates, par exemple des artères coronaires qui sont la cause des infarctus du myocarde et des lésions postischémiques, pour déclencher une action médicamenteuse.

La présente invention se propose de remédier à ces inconvénients et de fournir un dispositif de prévention des défaillances cardiaques brusques et de l'insuffisance cardiaque mécanique et qui permette de prévenir, dans un nombre élevé de cas, la survenue d'extrasystoles ventriculaires, de tachyarythmies ou tachycardies ventriculaires, et de fibrillations ventriculaires ainsi que la défaillance par insuffisance cardiaque ou thromboses ou hémorragies.

Un autre objectif de l'invention est de fournir un tel dispositif qui reste efficace dans une très grande diversité de situations cliniques y compris hémopathies et troubles de la coagulation sanguine.

Un autre objectif de l'invention est de fournir un tel dispositif qui puisse être réalisé à partir de constituants électroniques usuels utilisés depuis longtemps dans les appareils implantés.

Un autre objectif, encore, de l'invention est de fournir un tel dispositif qui perfectionne le stimulateur orthorythmique déjà connu.

Un autre objectif, encore, de l'invention est de fournir un tel dispositif facilement susceptible d'être associé, en combinaison, avec des dispositifs déjà connus et notamment stimulateurs cardiaques, défribrillateurs, dispositifs implantés d'assistance hémodynamique, dispositifs anti-ischémie, etc.

La présente invention a pour objet un dispositif de prévention de troubles cardiaques et sanguins, notamment des défaillances brusques, arythmies ventriculaires et insuffisances cardiaques, comportant :
- des moyens de détection de paramètres cardiaques et/ou sanguins,
- des moyens d'intervention cardiovasculaires et/ou d'alerte,
caractérisé en ce qu'outre des moyens pour la détection de la survenue de troubles cardiaque graves, il comporte :
- des moyens de mémorisation sensibles auxdits moyens de détection et susceptibles de garder, au moins temporairement, en mémoire, la suite des valeurs de paramètres correspondant à une séquence active de cycles cardiaques,
- et des moyens de comparaison, pour comparer ladite suite de valeurs de paramètres de ladite séquence active à des informations préalablement mémorisées de valeurs de paramètres, et, en cas de comparaison positive, pour déclencher la mise en oeuvre préventive desdits moyens d'intervention et/ou d'alerte.

Le dispositif selon l'invention peut être agencé, par exemple, à l'aide d'un programme, autour d'un microprocesseur relié à des éléments de mémoire. Ce moyen peut incorporer :
- les moyens logiques usuels pour l'acquisition des valeurs de paramètres à partir des capteurs qui les détectent ainsi que la mise en forme et, le cas échéant, le filtrage ; ces moyens sont déjà utilisés dans des dispositifs implantés pour détecter et analyser des signaux, par exemple électrocardiographiques ou d'autres paramètres tels que par exemple pression intravasculaire, concentration ionique ou chimique etc ;
- des moyens de comparaison, incorporant par exemple des valeurs de seuil auxquelles sont comparées des valeurs détectées de paramètres ;
- éventuellement des moyens logiques, également déjà usuellement utilisés et qui sont préprogrammés pour décider que le résultat de la comparaison constitue le diagnostic d'un trouble grave nécessitant la mise en oeuvre des moyens d'alerte et/ou d'intervention cardiaque ; ces moyens sont déjà habituellement utilisés dans les stimulateurs et dans les défibrillateurs cardiaques implantables.

Ces moyens qui, dans leur forme initiale étaient câblés et réalisés par des circuits électroniques discrets, sont maintenant couramment réalisés sous forme programmée en association avec un microprocesseur et son environnement usuel et ces dispositifs sont couramment mis dans le commerce.

Le dispositif comporte, en plus, et conformément à l'invention,
- des moyens permettant de mémoriser la suite des valeurs des paramètres correspondant à la séquence active de cycles cardiaques qui vient d'être détectée ;
- des moyens ayant mémorisé au moins une suite de valeurs antérieures constituant les informations préalablement mémorisées pour décider, le cas échéant, la mise en oeuvre préventive des moyens d'intervention et/ou d'alerte.

Bien entendu, ces moyens de comparaison peuvent être matériellement les mêmes, de même que les mémoires conservant les séquences actives ainsi que les informations préalablement mémorisées, peuvent être matériellement les mêmes que ceux précités, l'invention étant alors réalisée par la mise en oeuvre logique de ces moyens.

Dans un mode de mise en oeuvre particulièrement préféré, les moyens décrits ci-dessus sont pour tout ou partie, autoprogrammables de façon à permettre d'adapter le dispositif à l'évolution de l'état cardiovasculaire naturelle ou aux évolutions d'une part de prothèses placées au niveau du coeur et d'autre part des greffes tissulaires autologues ou hétérologues à cellules génétiquement modifiées ou non, d'une manière autonome par rapport à la programmation préétablie.

Cet auto-apprentissage qui peut être constitué en utilisant un logiciel usuel d'intelligence artificielle, peut être agencé pour pouvoir modifier notamment l'un au moins des éléments suivants, notamment sous leur forme logique :
- niveau de sensibilité des capteurs ainsi que logique d'identification de valeur de paramètres ;
- modification des informations préalablement mémorisées de valeurs de paramètre ou, tout en gardant les informations préalablement mémorisées, mémorisation d'autres informations acquises, par exemple les suites des valeurs de paramètres de séquences qui se sont réellement produites et qui, ayant précédé la survenue d'un trouble cardiaque, ont alors été mémorisées de façon permanente, de sorte que la séquence active de cycles cardiaques qui vient d'être détectée, sera comparée non pas à une série d'informations préalablement mémorisées mais à plusieurs séries d'informations préalablement mémorisées ;
- prise en compte de modifications provenant de l'activité propre du sujet par détection de cette activité;
- prise en compte d'une modification progressive ou rapide de l'état du muscle cardiaque, de la coagulation sanguine, et d'autres états physiologiques ;
- modifications du traitement automatique délivré par les moyens d'intervention cardiaque, dans leur intensité, leur séquence ou dans la nature de drogues automatiquement délivrées.

Par séquence active de cycles cardiaques, dans le sens de l'invention, on entend une séquence de cycles cardiaques, de préférence directement consécutifs qui vient de se terminer, ou qui précède de peu l'instant de ladite comparaison.

Ainsi, de préférence, la séquence active de cycles cardiaques est la séquence constituée par les n derniers cycles cardiaques qui viennent de se produire au moment considéré, le nombre n étant de préférence compris 6 ou 8 et quelques centaines ou milliers. Cependant, on peut également utiliser des séquences de très longue durée, de l'ordre de plusieurs heures à plusieurs jours, soit de dizaines de milliers à des millions de cycles. Ainsi, par exemple, dans une forme de réalisation préférée, la séquence active est la séquence des 72 derniers cycles qui viennent de se produire. Dans une telle réalisation, chaque fois qu'un nouveau cycle cardiaque est détecté, ce nouveau cycle fait partie de la séquence active et le cycle le plus ancien en est éliminé de façon que n reste constant.

Cependant, quoique cela ne soit pas préféré, la séquence active pourrait également être constituée d'un nombre non constant de cycles, par exemple tous les cycles qui se sont produits pendant une durée déterminée, par exemple comprise entre 30 secondes et 120 minutes, avant l'instant de la comparaison.

En variante encore, la séquence active peut être constituée d'une séquence de cycles qui ne se termine pas immédiatement par le cycle en cours mais qui se termine antérieurement au cycle en cours à l'instant déterminé.

Conformément à l'invention, lesdits moyens de mémorisation gardent en mémoire l'ensemble de valeurs ordonnées correspondant au(x) paramètre(s) mesuré(s) pendant la séquence active.

Par paramètre cardiaque, dans le sens de l'invention, on entend tout paramètre détectable, mesurable ou calculable donnant une information sur l'état cardiovasculaire et/ou sanguin.

Ces paramètres peuvent être de type électrique, c'est-à-dire dont l'acquisition est susceptible d'être faite par des moyens tels que des électrodes ou capteurs notamment, cutanées, sous cutanées, juxtacardiaques, intracacavitaires, intra-myocardiques, péricardiques ou intravasculaires. Parmi ces paramètres, on préfère notamment les suivants :
- période et fréquence cardiaques, cycle par cycle, par exemple intervalle RR,
- forme de l'électro-cardiogramme,
- vectocardiogramme,
- complexes QRS notamment amplitude, morphologie,
- intervalle de l'onde T (par exemple valeur de QT),
- forme et/ou propagation de l'onde T,
- gradient de l'onde T, vectocardiogramme,
- détection de potentiel QRS retardé,
- impédances endocavitaires, ventriculaires ou auriculaires, impédances transpariétales, intra-veines caves, intra-canal thoracique.
- agrégation et activation plaquettaire (déterminée par exemple par détection optique),
- activation sélective des lymphocytes (liaisons antigène-anticorps)
- facteurs coagulants et/ou anticoagulants, de fibrinogenèse et de fibrinolyse.

Les valeurs mémorisées de ces paramètres, à chaque cycle, peuvent être les valeurs effectivement mesurées ou détectées d'un paramètre. Cependant, on comprend que l'on peut également, à partir des valeurs mesurées, créer des valeurs calculées en fonction de plusieurs paramètres ou de l'analyse d'un paramètre complexe. En particulier, lorsque l'on utilise l'un des paramètres relatifs à l'onde T, on peut utiliser le rapport de l'intervalle QT du cycle en cours à la valeur du cycle RR, par exemple immédiatement précédent, en cours ou à venir.

Lesdits paramètres dits électriques peuvent, dans une réalisation extrêmement simple, être détectés entre deux points électriques, par exemple entre une électrode intracavitaire droite et une électrode portée à la surface du boîtier d'un dispositif implanté selon l'invention. Cependant, on préfère, dans l'invention, détecter les paramètres, par exemple l'électrovectorcardiogramme, par mesure entre au moins trois points de référence, d'une façon en soi connue, et ceci notamment dans le cas où l'on utilise comme paramètre la durée, la forme ou l'amplitude des complexes ou ondes QRS, ou T, ou le décalage, l'orientation et la dispersion de ces ondes suivant les différentes directions géométriques du système de référence de mesure.

La détection peut être effectuée d'une façon originale qui ne trace pas la simple résultante des vecteurs électriques des ventricules gauche et droit mais au contraire respecte séparément les parcours vectoriels de ou dans chaque ventricule à chaque cycle afin de pouvoir détecter les propagations et intervalles critiques de chaque ventricule. A cette fin, on utilise au moins une électrode endocavitaire dans le ventricule droit et de préférence également dans le ventricule gauche de préférence en situation pariétale. De cette façon, les électrodes endocardiques isolées menues branchées en monopolaire par rapport à des électrodes épicardiques, juxtacardiaques ou sous-cutanées permettent l'enregistrement des potentiels endocardiques.

A l'inverse, ce dispositif, selon une variante de l'invention, permet de commuter simultanément ou successivement, les électrodes endocardiques en position de continuité électrique entre elles et avec le milieu sanguin (par réunion électrique avec le boîtier métallique du stimulateur cardiaque et une électrode auriculaire par exemple) et de commuter simultanément les électrodes isolées épi- ou juxtacardiaques ou cutanées en position monopolaire. De cette dernière façon, on obtient les potentiels épicardiques. L'acquisition simultanée ou successive alternée des potentiels épicardiques et endocardiques sur les mêmes axes ou vecteurs permet une mesure très précise des gradients transpariétaux AQRS et AT ; ceci ouvre le domaine de la détection e.c.g. très précoce des ischémies ou autres lésions myocardiques même légères et silencieuses avec leur localisation, nécessaire pour la prévention automatique des défaillances cardiaques par polygraphies internes analysées et intervention sélective instantanée.

Le dispositif peut aussi mettre en oeuvre des tests électrophysiologiques utilisés jusqu'à présent pour provoquer des tachycardies ventriculaires après extrastimuli programmés, et ceci par des extrastimuli avec des intervalles de couplage orthorythmiques (en pourcentage ou en millisecondes) pour analyser les réactions polygraphiques cardio-vasculaires, d'hémostase et autres (notamment e.c.g., durées, gradients, et dispersions QT et AT, QRS, mécanogrammes cardiovasculaires et autres paramètres).

Le dispositif peut comporter, en mémoire, des informations programmées ou, en variante, acquises pendant le fonctionnement sur les modifications normales des paramètres à l'effort, à l'émotion et au retour au repos, ces modifications ne devant pas être confondues avec des modifications pathologiques. Cette identification s'effectue par des moyens de détection multiples de l'effort déjà connus dans les stimulateurs cardiaques de type VVIR et mémorisation dans un registre spécial.

D'autres paramètres électriques mesurés ou calculés peuvent être :
- nombre ou pourcentage, et morphologie des extrasystoles ventriculaires, ainsi que leur intervalle de couplage RR',
- surface, obtenue par intégration , des complexes QRS et/ou des ondes T,
- durée ou forme des ondes T.

On préfère, surtout dans le cas où l'on effectue des détections sur plusieurs axes géométriques de façon à pouvoir utiliser comme paramètres, non seulement les valeurs absolues électriques mesurées mais également les décalages temporaires qui peuvent se produire sur les différents axes, déterminer par un moyen en soi connu la direction de l'axe électrique du coeur au moment de la mesure. On sait en effet que l'axe électrique du coeur, qui peut varier de façon pathologique en cas d'atteinte cardiaque, varie également temporairement en fonction du cycle respiratoire ou de la situation posturale de la personne connectée au dispositif selon l'invention. L'acquisition instantanée de la direction de l'axe électrique du coeur peut être obtenue, par exemple, par comparaison des signaux obtenus sur différentes dérivations, par exemple D1, D2, D3 du coeur.

On comprend cependant que d'autres paramètres cardiaques, sanguins ou cardiovasculaires peuvent être détectés et utilisés dans l'invention, par exemple des paramètres chimiques tels que saturation en oxygène ou en CO₂, pH, acide lactique, myosine alpha ou bêta, catécholamines, endothéline, concentration en ions tels que K⁺, Ca⁺⁺, Na⁺, teneur en certains facteurs coagulants, anticoagulants, fibrinogéniques ou fibrinolytiques, teneur du myocarde en glutathion ou autres anti-oxydants, en radicaux libres toxiques ou des paramètres mécaniques tels que durée ou rapports de la concentration isovolumétrique (PEP), diagramme-pression ou pression-volume, volume du coeur, valeur de la pression d'éjection, dérivée dP/dt de la pression, intégrale de la pression (pression.temps) par cycle, etc.

Ainsi le dispositif peut prévenir l'installation d'une thrombose ou hémorragie vasculaire en utilisant des moyens capables de détecter in situ l'état fonctionnel de la coagulation sanguine, en utilisant par exemple un dispositif décrit par l'inventeur dans EP-A-0 348 271 et d'injecter immédiatement à l'aide de pompes implantables connues des substances, par exemple les inhibiteurs de thrombine à petites molécules tels par exemple N-méthyl-phénylglycyl-pro-arginal 0,5 mg/kg et/ou hirudine 0,25 mg/Kg, et/ou Aspirine 0,02 g/kg ou encore un inhibiteur du facteur XIII, tel qu'un dérivé de l'iodacétamide (par exemple fluoracétamide, 1 mg). Une telle inhibition de la thrombine, sans danger d'hémorragie, peut être suffisante pour potentialiser suffisamment la tPA intrinsèque naturelle pour dissoudre le caillot en formation ; si cela n'est pas le cas, le détecteur de la coagulation sanguine de l'inventeur enregistre la nouvelle vague de fibrinolyse spontanée et l'appareil peut alors injecter automatiquement une dose très faible d'un thrombolytique.

Après sa dissolution ou stabilisation à minima de la thrombose un retour de celle-ci pourra être évité par l'alarme sélective donnée par la télécommande du dispositif implanté qui avertit le porteur et conseille la prise d'un anti-coagulant adapté.

Le dispositif intravasculaire de détection permet de connaître le début de la formation de fibrine dans un autre vaisseau en utilisant l'équivalent bio-chimique (EP-A-0 348 271) d'une petite plaie permanente autour de laquelle la balance fibrinolyse, fibrinogenèse bascule vers une fibrinolyse intense et rapide observable et caractéristique d'une thrombose d'une certaine importance. Selon que la thrombose est dissoute ou non la fibrinolyse s'arrête ou se poursuit, permettant de distinguer l'arrêt ou non de la thrombose, de le signaler à un moniteur et de commander la poursuite ou non de l'administration automatique d'un médicament.

De façon avantageuse, le dispositif peut comporter un capteur optique implanté, tel qu'une microcaméra éclairante, orientable et nettoyable, actuellement disponible, en position intracardiaque et capable de visualiser une partie du coeur à l'échelle cellulaire, intercellulaire et infra-cellulaire.

Afin de mesurer avec précision les mouvements et dimensions des parois et cavités cardiaques, le dispositif peut également comprendre une sonde intracardiaque miniature orientable d'émission et réception ultrasonore dont les signaux sont traités par le microcontrôleur implanté.

Un autre paramètre intéressant peut être la valeur de la pression artérielle, qui peut être mesurée, par exemple par amplification des vibrations spontanées sonores ou infrasonores aux fréquences de résonance de l'arbre artériel. Le cas échéant, ces vibrations peuvent être provoquées par émission sonore endocavitaire à basse fréquence ou impulsion sonore dont les déformations et l'amortissement sont proportionnels à la pression dans l'arbre artériel.

Dans une variante, l'analyse de la fréquence de résonance, notamment du bruit systolique détecté sur ou près d'une paroi ventriculaire, permet la surveillance de sa fonction contractile.

La détection des fréquences de résonance des prothèses cardiaques valvulaires, myocardiques, artérielles ou aortiques permet la surveillance de ces prothèses.

Les valeurs de paramètre peuvent être acquises à chaque cycle de la séquence active, ou seulement à certains cycles, par exemple 1 cycle sur 10 ou selon une autre sélection, par exemple 1 cycle sur 500, ces sélections pouvant d'ailleurs être différentes d'un paramètre à l'autre dans le temps, selon l'état de détection de chaque paramètre et d'un paramètre à l'autre à l'intérieur d'une même séquence.

Bien entendu, le dispositif peut être agencé pour procéder à des tests d'exploration ou de diagnostic en provoquant des variations des paramètres, par exemple par stimulation électrique à intervalle programmé (par exemple recherche d'inhibition d'extrasystoles ventriculaires par accélération légère du rythme de stimulation), ou par action de produits pharmacologiques, chimiques ou biologiques.

Les résultats de ces tests peuvent alors avantageusement être utilisés dans le cadre d'un progrmame d'autoapprentissage, soit pour des modifications de la logique de comparaison en fonction des tendances évolutives des paramètres pathologiques ou physiologiques, soit pour des modifications du traitement, par exemple variation d'intensité des stimulations électriques ou des doses de médicaments.

Par moyen d'intervention cardiaque dans le sens de l'invention, on entend tout moyen permettant de prévenir l'apparition d'extrasystoles, de tachycardies ou tachyarythmies ventriculaires, ou de défaillances brusques ou d'une insuffisance mécanique. Ces moyens peuvent comprendre des moyens implantés tels que des micropompes, permettant de délivrer une drogue, par exemple dans le sang ou dans le myocarde ou une assistance hémodynamique partielle ou totale, par exemple, prévue dans la demande de brevet EP-A-0.348.271. Cependant dans la forme de réalisation la plus simple, ces moyens peuvent être des moyens de stimulation électrique d'un type classique permettant l'envoi d'une ou de plusieurs impulsions de stimulation avec un potentiel éventuellement élevé, suffisant pour resynchroniser les cellules du myocarde.

A titre d'exemple les moyens d'intervention peuvent comprendre des électrodes périphériques, par exemple sous-cutanées, en deux points opposés du thorax pour générer des impulsions de stimulation électrique, infraliminaires pour le muscle cardiaque, mais suffisantes pour stimuler le système neurovégétatif, notamment en cas de stabilité excessive de la période RR dans la séquence active éventuellement associée à une diminution des réactions mécaniques à l'effort ou après injection de substance hypertensives.

Les moyens de comparaison dans le sens de l'invention sont des moyens de comparaison de type logique et de préférence implantables, articulés par exemple autour d'un microprocesseur.

Dans une première forme de réalisation de l'invention, le dispositif selon l'invention conserve en mémoire les valeurs des paramètres d'au moins une séquence ancienne dont on sait qu'elle a été suivie d'un incident cardiaque, par exemple bradycardie, tachycardie ventriculaire ou tachyarythmie ventriculaire, fibrillation ventriculaire, arrêt cardiaque, ou bien dont le médecin pense qu'elle est associée à un risque de défaillance mécanique. Ces valeurs peuvent avoir été introduites une fois pour toutes dans l'appareil et mémorisées dans celui-ci mais, dans un mode de mise en oeuvre perfectionné, ces valeurs peuvent avoir été et continuent à être acquises automatiquement par le dispositif selon l'invention, qui est alors agencé pour conserver à demeure en mémoire la suite des valeurs des paramètres de la séquence de n (par exemple 72) cycles qui a été suivie, immédiatement, ou de préférence, à distance par l'incident, par exemple la tachycardie ou la fibrillation ou défaillance purement mécanique du myocarde ou une brusque fibrinolyse ou fibrinogenèse. Dans un tel mode de mise en oeuvre, l'appareil est agencé pour, non seulement mémoriser la séquence active, mais également conserver, indéfiniment ou non, l'ancienne séquence active qui a précédé directement ou non l'incident.

Des moyens sont prévus pour annuler des séquences critiques anciennes par exemple après traitement pharmacologique, génétique ou chirurgical.

Conformément à l'invention, lesdits moyens de comparaison comparent la suite des valeurs de paramètres de la séquence active dont ils viennent de faire l'acquisition, à la ou aux suites de valeurs des anciennes séquences dangereuses mémorisées automatiquement ou programmées. En cas de similitude entre ces suites comparées, l'appareil déclenche l'activation des moyens d'intervention, par exemple stimulation électrique, et/ou une alarme reçue sur un récepteur extracorporel.

Par similitude, dans le sens de la présente invention, on entend un certain degré de ressemblance entre les paramètres des deux séquences et on peut se référer ici à des techniques voisines des techniques connues d'analyse d'images permettant de décider qu'une image ressemble à une autre à partir d'un certain degré de similitude sans qu'une coincidence parfaite soit indispensable. Ainsi, à titre d'exemple, si le paramètre est constitué simplement par la mesure du rythme cardiaque, on peut concevoir que le dispositif sera agencé pour qu'il considère qu'il y a similitude non seulement en cas de similitude à un pourcentage prédéterminé près, par exemple 4 %, des valeurs absolues des différents cycles mais également en cas de ressemblance de type analogique ou homothétique des deux séquences, ou encore de parties significatives de ces séquences.

Conformément à l'invention, et notamment dans le cas où l'appareil est agencé de façon à détecter et mémoriser lui-même les séquences dangereuses qui ont effectivement précédé un incident, on préférera ne pas déclencher l'intervention au tout dernier moment, mais au contraire avant. Ceci peut être fait, par exemple, dans le cas où n égale 36, en conservant en mémoire, par exemple, une séquence de 72 cycles immédiatement antérieurs à la survenue de l'incident et en comparant la séquence active aux 36 premiers cycles de la séquence dangereuse mémorisée.

Dans une deuxième forme de réalisation de l'invention, applicable notamment, mais non exclusivement, au cas où l'un des paramètres principaux est relatif à l'onde T, on compare la suite de valeurs du paramètre de la séquence active, non pas à une suite de valeurs d'une séquence ayant précédé un événement dangereux, mais au contraire à des informations relatives à des valeurs ou des évolutions considérées comme normales chez le patient et l'on décide que la comparaison est positive, c'est-à-dire déclenche l'alerte ou l'intervention des moyens par exemple de stimulation, lorsque les valeurs de paramètres de la séquence active diffèrent significativement de ladite information. Ainsi, par exemple, si la suite de valeurs de paramètre est constituée par le rapport QT/RR (RR pouvant être la durée d'un cycle de l'intervalle QT en question, ou bien en variante, la durée d'un cycle précédent), on détermine, soit avant la mise en place de l'appareil, soit directement par l'appareil lui-même, la fonction, normale chez le patient, des variations dudit rapport QT/RR et on procède à la stimulation si, lors de la séquence active, la courbe mesurée de cette fonction diffère d'au moins une certaine grandeur ou pourcentage prédéterminé, de la courbe habituelle chez le patient.

Bien entendu, les première et deuxième formes de réalisation de l'invention peuvent être combinées dans le même dispositif.

Quelle que soit la forme de réalisation, l'appareil peut encore comporter des moyens de comparaison sensibles instantanément à une variation brusque, c'est-à-dire pratiquement instantanée, d'un paramètre pour déclencher une intervention immédiate. Par exemple, l'appareil peut incorporer tout ou partie des moyens du dispositif orthorythmique précédemment décrit dans les brevets US précités.

D'une manière générale le dispositif est agencé pour discriminer sur des mémoires séparées les valeurs de paramètres selon qu'ils proviennent d'un rythme spontané, électrostimulé ou fusionné.

Par exemple, une diminution importante et instantanée de la longueur du cycle RR sera considérée comme une extrasystole et une intervention de stimulation se produira immédiatement après un intervalle programmé. Ou bien encore, par exemple, une modification rapide du décalage QT dans l'une des dérivations électriques de détection de l'électrocardiogramme par rapport à une autre dérivation (par exemple V1, V5) pourra provoquer une réponse immédiate du dispositif durant le même cycle ou non.

Le dispositif selon l'invention est de préférence du type implantable et, dans ce cas, on préfère qu'il comprenne, outre les moyens de détection et d'acquisition des valeurs de paramètres, lesquels peuvent éventuellement être d'un type classique, et les moyens d'intervention de stimulation, qui peuvent également éventuellement être de type classique, des moyens de mémorisation d'un type quelconque et une unité centrale effectuant les calculs et comparaisons et gérant le fonctionnement de l'ensemble, par exemple adaptation aux changements physiologiques du type repos-effort, telle que par exemple, un microprocesseur. Le dispositif, s'il est implanté, comporte encore une source d'énergie, par exemple une pile électrique.

Le dispositif selon l'invention, lorsqu'il est implanté, peut comporter avantageusement des moyens de télétransmission depuis et vers le dispositif implanté. Ces moyens sont déjà actuellement utilisés, dans les stimulateurs implantés et n'ont pas à être détaillés. Ils sont agencés pour permettre la télécommande ou téléprogrammation, la télé-alarme et/ou la télésurveillance, avec de préférence télé-affichage sur un moniteur, notamment pour surveiller le bon fonctionnement des capteurs de paramètres et du microprocesseur, procéder à des tests automatiques d'efficacité diagnostique et thérapeutique, reprogrammer de nouvelles définitions de séquence active, de critères de comparaison, et de tendances évolutives. Ils peuvent également être utilisés pour transmettre les réactions à des tests surveillant l'effet de médications chimiques ou autres.

En plus, les moyens de télécommande peuvent être agencés pour pouvoir commander depuis l'extérieur l'administration intravasculaire automatique et immédiate de doses préétablies de médicament (trinitrine par exemple), y compris par le patient lui-même et, en cas de persistance de douleur, l'administration automatique d'inhibiteurs de la thrombine ou de faibles doses de fibrinolytiques ou autres. Ainsi le dispositif peut comprendre, en association ou tout à fait indépendamment du reste de l'invention, des moyens implantables de distribution de très faibles doses d'au moins un médicament dans la circulation sanguine, par exemple une pompe à commande automatique. De préférence, ce dispositif permet la délivrance d'inhibiteurs de la thrombine, notamment de petits peptides tels que (D) N-méthyl-phénylglycyl-pro-argynal ou d'autres produits similaires, ou encore d'hirudine. La dose d'un inhibiteur de peptide court de ce genre est de préférence de l'ordre de 0,1 à 1 mg par kg corporel. De préférence le dispositif peut également comporter un moyen d'administration d'un vasodilatateur coronaire tel que la trinitrine ou ses dérivés pour une administration d'une très faible dose, par exemple de l'ordre de 10 mg. De préférence, le dispositif peut comporter, outre le moyen d'administration d'inhibiteur de la thrombine, une source de délivrance de dose d'aspirine et/ou une source de délivrance d'un fibrinolytique à très faible dose tel que t-PA, par exemple en dose unique de 10 à 20 mg. Le boîtier de télécommande, notamment lorsqu'il est destiné à être utilisé par le porteur du dispositif lui-même, peut avantageusement comprendre plusieurs commandes, telles que par exemple des boutons, le cas échéant associés à un système de déverrouillage, de préférence une commande ou poussoir par type de médicament. Pour des patients à risque de thrombose coronarienne, le dispositif à action manuelle peut avantageusement être prévu pour imposer l'ordre suivant : administration volontaire de trinitrine suivie d'une durée d'attente de quelques minutes, - administration volontaire d'une dose d'inhibiteur de la thrombine, suivie d'une durée de plusieurs minutes, puis, dans l'hypothèse où cette source est présente, administration volontaire de la très petite dose de fibrinolytique. On peut prévoir que le dispositif autorise la délivrance volontaire à un intervalle minimum de plusieurs minutes, de trinitrine ou d'inhibiteurs de la thrombine. Ainsi, le patient sera contraint de commencer, en cas d'apparition de douleurs, par l'administration d'au moins une dose de trinitrine puis, si la douleur ne cède pas, l'administration d'une dose d'inhibiteur, puis, si la douleur persiste et si l'appareil le comporte, l'administration d'une dose de fibrinolytique. Pour des malades non coronariens susceptibles de thromboses non coronarienne, par exemple thrombose pulmonaire ou cérébrale, le dispositif de distribution de trinitrine ne sera pas présent ou approvisionné.

De préférence, ce dispositif est associé à des moyens d'acquisition et d'interprétation de l'électrocardiogramme qui peuvent être d'un type tout à fait classique, utilisant un microprocesseur pour analyser l'électrocardiogramme et détecter l'existence d'une variation pouvant traduire une thrombose coronarienne ou une embolie pulmonaire de sorte que l'administration de l'inhibiteur ou du fibronolytique ne sera autorisée qu'après détection positive électrocardiographique de cette thrombose. Dans la forme de réalisation préférée, le dispositif est associé aux autres moyens décrits dans l'invention permettant la prévention des troubles cardiaques et/ou sanguins, y compris la surveillance permanente des facteurs de coagulation.

Ainsi, par exemple, le médecin pourra procéder à la mesure classique de la tension artérielle par brassard et obtenir les valeurs, à cet instant, de paramètres multiples correspondant à cette tension, notamment dp/dt, PEP, intégrale QRS, intégrale du segment ST, volume systolique, volume diastolique, impédance transpariétale du myocarde, etc... En mesurant des tensions de valeurs différentes, éventuellement provoquées, à des instants différents, on peut ainsi réaliser un abaque de tension artérielle, qui, mémorisé dans le dispositif, permettra à chaque instant un calcul automatique de cette tension qui formera l'un des paramètres utilisés dans l'appareil.

Dans une variante de la mesure d'une pression artérielle, on peut également utiliser l'analyse fine de la forme de la courbe de pression intraventriculaire qui est également un reflet de la pression.

Le dispositif peut également être externe et, dans ce cas, il peut comprendre un ordinateur d'un type quelconque avec éventuellement des périphériques tels que clavier, écran et imprimante, ainsi que les moyens de détection, et de stimulation avec les interfaces analogiques/numériques requises, des moyens de couplage opto-électroniques pouvant être interposés, pour la sécurité électrique du sujet entre les parties connectées à l'organisme telles que capteur, électrodes et moyens de stimulation et le reste du dispositif.

L'invention a également pour objet le procédé de traitement du coeur consistant à détecter des paramètres cardiaques ou sanguins, à mémoriser, au moins temporairement, la suite des valeurs de paramètres correspondant à une séquence active de cycles cardiaques, à comparer ladite suite de valeurs de paramètres, à une information préalablement mémorisée de valeurs de paramètres, et, en cas de comparaison positive, à déclencher la mise en oeuvre de moyens d'alerte ou d'intervention cardiaque ou sanguine.

La prévention efficace des défaillances cardiovasculaires peut prévoir une adaptation constante à l'évolution des possibilités thérapeutiques nouvelles. Celles-ci comportent A- les greffes tissulaires cardiaques partielles ou totales et B- les coeurs artificiels partiels ou totaux et C- le traitement des substrats pathologiques.
A - Greffes tissulaires de tissus cardiaques prélevés du patient et réparés génétiquement, ensuite multipliées in vitro et contrôlées avant réimplantation sélective dans le coeur du patient où ses cellules rénovées assurent progressivement, par leur croissance, la réparation du coeur. Dans certains cas, la réparation génétique pourra être réalisée par simple injection de gènes exogènes capables d'obtenir leur expression par transfert de gène, par exemple gène de la myosine cardiaque.
B - Greffe intracardiaque de tissu cardiaque naturel étranger, par exemple embryonnaire naturel ou transgénique ; dans ce dernier cas, des chromosomes ou groupes de gènes des cellules embryonnaires sont enlevés et remplacés, par exemple par des éléments génétiques correspondants du sujet et par exemple renforcés par des gènes anti-infection afin d'améliorer la tolérance et le fonctionnement, après multiplication in vitro, du tissu greffé qui se multipliera et remplacera progressivement in situ les cellules cardiaques anciennes malades ou vieillies.
C - La reconstitution in vitro d'un coeur complet ventriculaire sur mesure à partir de tissus autologues ou homologues génétiquement réparés, améliorés et contrôlés, éventuellemnet associés à une armature ou des structures mécaniques.

Ces greffes ou reconstitutions tissulaires partielles ou complètes utilisant des cellules génétiquement réparées remplaceront vraisemblablement progressivement les greffes cardiaques classiques dont la survie est souvent remarquable mais limitée dans le temps ne serait-ce que par l'athérosclérose intracardiaque et les traitements immunosuppresseurs.

L'invention, dans une variante, s'applique à ces coeurs soumis à la reconstitution par cellules réparées qui pourront avoir des troubles du rythme graves ou des défaillances par insuffisances mécaniques ou excès de leur hémodynamique. Dans ces cas, l'invention utilisera les détection multiples cardio-vasculaires analysées dont le logiciel sera programmé et se programmera lui-même en tenant compte du fonctionnement et de l'évolution normale du coeur réparé.

L'utilisation de coeurs artificiels implantés partiels ou totaux (dont la première expérimentation avec survie de l'animal a été publiée par l'auteur avec Coraboeuf et Monod et al. C. R. Soc. Biol. janvier 1956, T:150, pages 48-51) sollicitera une variante de la présente invention car un coeur artificiel, qu'il soit réglé à un rythme, volume ou pression systolique constant ou non, s'adaptant à l'effort, provoquera une réaction de l'arbre artériel et veineux du système neurovégétatif et une hypercoagulabilité sanguine. En cas de danger de thrombose détectée une injection préventive automatique ou déclenchée par télécommande d'inhibiteur de thrombine peut être réalisée. Dans ces conditions, afin d'éviter un "collapsus ou désamorçage" du coeur artificiel par dilatation trop forte des capillaires et des veines ou des spasmes artériels dangereux, la surveillance et la régulation préventive d'un coeur artificiel deviennent une nouvelle application de l'invention. Les paramètres mesurés seront préférentiellement les pressions veineuses centrales et artérielles et, en cas de détection de baisse de pression veineuse, le dispositif adresse une instruction de commande au coeur artificiel, par exemple pour réduire le débit et éviter le collapsus ou administrer une drogue telle que l'adrénaline.

Une variante de l'invention s'applique aux coeurs dont l'état général est encore satisfaisant mais dont une partie seulement constitue un substrat très pathologique, par exemple une ischémie, nécrose ou dégénérescence fibroblastique localisée produisant ralentissement et déviation de l'activation et repolarisation myocardique, source de tachycardies ventriculaires. Dans ce cas, le dispositif de l'invention détectera et localisera le substrat malade dans le myocarde sain. Cette détection peut être avantageusement effectuée par détection des modifications électrocardiographiques, par modification de l'aspect régional des ondes T et de la durée QT et du gradient AT, ainsi que d'une anomalie mécanique (augmentation du PEP et diminution de dp/dt, du volume systolique et de la fraction d'éjection FE). Le dispositif pourra déclencher soit une alarme sur télémoniteur avec indication des paramètres détectés du substrat malade en affichant, par exemple ischémie locale gauche antérieure, besoin d'intervention, soit procéder lui-même à l'aide d'une mini-pompe implantée à la perfusion sanguine, par exemple de certains facteurs de croissance fibroblastiques (FGB) capables de déclencher une revascularisation et une réimplantation de cellules myocardiques normales dans la zone ischémiée ou nécrosée.

On peut aussi injecter dans le substrat malade par cathétérisme des cellules génétiquement capables de revasculariser et de recoloniser le myocarde.

L'invention s'applique aussi, de façon avantageuse, aux coeurs portant des prothèses valvulaires, vasculaires ou ventriculaires ainsi qu'aux coeurs transplantés ou recevant des greffes tissulaires.

De préférence, dans tous ces cas, le dispositif incorporera un programme logique d'intelligence artificielle de façon à adapter son fonctionnement à l'évolution physiologique ou physiopathologique. Par exemple, ceci pourrait être effectué par détection et évaluation connue de la fonction systolique et diastolique qui permettrait, par exemple, de modifier progressivement les moyens de comparaison.

D'autres avantages et caractéristiques de l'invention apparaîtront à la lecture de la description suivante, faite à titre d'exemple non limitatif et se référant aux dessins annexés dans lesquels :
la figure 1 représente une vue sous forme de bloc schéma fonctionnel d'un dispositif implantable selon l'invention.

Les figures 2 et 3 représentent des courbes de valeurs de paramètres utilisées dans le dispositif.

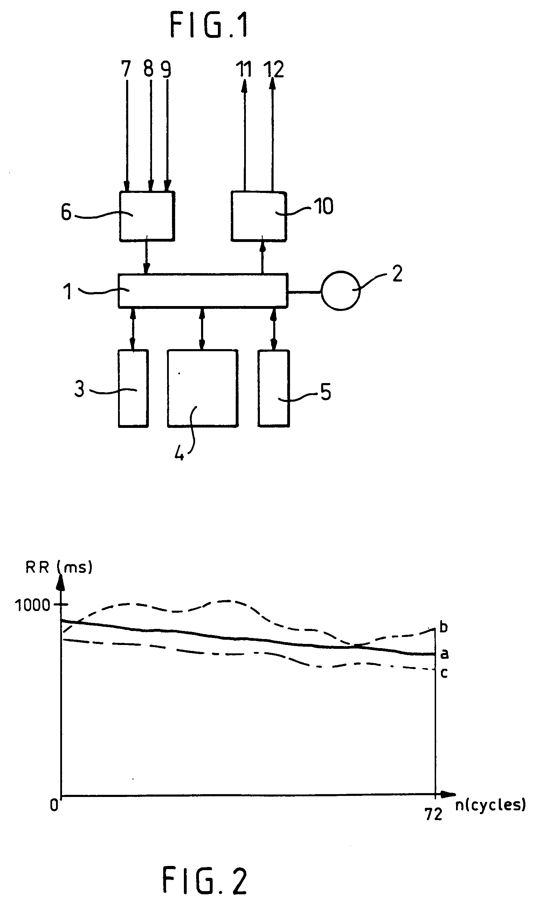

Le dispositif implantable selon l'invention, représenté sur la figure 1, comporte une unité centrale 1 telle qu'un microprocesseur, à laquelle sont adjoints une source de courant électrique 2 et des mémoires telles qu'une mémoire 3, de préférence de type RAM, destinée à mémoriser la séquence active en cours, une mémoire 4, de préférence de type RAM, destinée à mémoriser les suites de paramètres de plusieurs séquences, par exemple une séquence entrée par programmation, et une ou plusieurs séquences qui peuvent se produire et qui sont suivies d'un incident cardiaque détecté par le dispositif, ainsi qu'une mémoire 5, de préférence de type ROM comprenant des instructions pour le fonctionnement général du microprocesseur 1. D'autres mémoires additionnelles peuvent comprendre éventuellement d'autres informations telles que par exemple des informations relatives au patient, à sa maladie et son évolution, aux modifications physiologiques ainsi que des informations relatives au type d'intervention, par exemple intensité, puissance, et localisation et orientation des stimulations électriques, etc. Le microprocesseur 1 est d'autre part relié à une unité de conversion analogique/numérique 6 à laquelle aboutissent, par des conducteurs implantés convenables, les informations de différents capteurs 7, 8, 9 tels qu'électrodes de détection, capteurs de pression, etc. Enfin, l'unité centrale 1 est reliée à une logique de stimulation 10, par exemple d'un type connu, capable de procéder au traitement du coeur, par l'envoi d'impulsions de stimulation, actionnant des effecteurs tels que 11, 12, par exemple des électrodes de stimulation. L'ensemble des composants 1-6, 10 est contenu dans un boîtier étanche du type de ceux utilisés dans les stimulateurs cardiaques implantés qui peut lui-même porter, par exemple, une électrode aboutissant au convertisseur 6.

### Exemple 1

On a représenté sur la figure 2, pour un patient déterminé, la courbe de valeurs de paramètres d'une séquence que le médecin a estimée dangereuse pour le patient à la suite d'une étude sur enregistrement HOLTER.

Le paramètre est la période RR du cycle cardiaque. La courbe a sur la figure 2 représente l'évolution de la période des cycles cardiaques pendant 72 cycles consécutifs. Comme on le voit, cette évolution se caractérise d'une part par une grande constance du rapport RR dans laquelle les intervalles RR de deux cycles consécutifs diffèrent de moins de 5%, ainsi que par une tendance générale à une diminution très progressive de la durée de l'intervalle RR. La courbe ainsi constituée est mémorisée dans la mémoire 4.

12 cycles après la fin de la séquence représentée, le patient avait fait une tachycardie ventriculaire détectée sur HOLTER.

Le microprocesseur maintient constamment dans la mémoire 3, la courbe analogue b constituée par les valeurs des intervalles RR des 72 derniers cycles détectés, c'est-à-dire de la séquence active.

A chaque cycle, le microprocesseur 1 compare la courbe b de la séquence active qui vient d'être remise à jour dans la mémoire 3 à la courbe a enregistrée dans la mémoire 4.

Ainsi, par exemple la courbe active b, représentée sur la figure 2, diffère de la courbe a par des variations de rythme parfois supérieures à 5 %, ainsi que par des fluctuations d'accroissement et de diminution. Cette séquence n'est pas considérée comme dangereuse.

En c, on a représenté une séquence considérée comme dangereuse et qui se caractérise à la fois par une tendance à l'accélération et par de très faibles écarts, inférieurs à 5 % entre deux intervalles RR consécutifs.

Dans le cas où cette séquence active de 72 cycles survient, la comparaison avec la séquence a est positive et le microprocesseur 1 commande alors l'envoi d'une série d'impulsions de stimulation par le dispositif 10, à un rythme légèrement supérieur au rythme spontané et avec une puissance de quatre fois le seuil.

### Exemple 2

La séquence active comporte cette fois 1152 cycles.

Le dispositif utilisé dans cet exemple comporte une pluralité d'électrodes endocavitaires et sous-cutanées agencées, d'une façon en soi connue, de façon à obtenir l'électrocardiogramme sur plusieurs dérivations géométriques. Le microprocesseur est programmé pour déterminer, à partir des signaux reçus sur les différentes électrodes, les paramètres suivants :
- intervalle RR (a1),
- détection et comptage des extrasystoles ventriculaires identifiées par leur morphologie, durée et propagation,
- intervalle de couplage RR' des extrasystoles, c'est-à-dire intervalle entre une extrasystole et le complexe QRS antérieur (a3),
- pourcentage (a2) des extrasystoles ventriculaires,
- détection de l'onde T et durée de l'intervalle QT (a4),
- dispersion (a5) de l'intervalle QT, c'est-à-dire différence, dans un même cycle, entre les durées des intervalles de QT dans une dérivation ventriculaire gauche par exemple antérieure par rapport à la pariétale et la postérieure.

Le dispositif comporte encore un capteur de pression intra-ventriculaire droit permettant d'obtenir la valeur du PEP (a6) et également, par le microprocesseur, de calculer la variation de la pression dp/dt (a7).

Il comporte également des moyens logiques calculant la surface du mécanogramme de pression (a8), un détecteur de pression partielle d'oxygène (a9), et un capteur endocavitaire de pression artérielle (a10).

Conformément à l'invention, le dispositif mesure et calcule ces différents paramètres à chaque cycle qui se produit et conserve les valeurs des 1152 derniers cycles, c'est-à-dire de la séquence active, dans la mémoire 3.

En fait, dans l'exemple représenté, le dispositif conserve dans la mémoire 3 la séquence des valeurs de paramètres des 9216 derniers cycles, ce qui inclut bien entendu les 1152 derniers cycles.

Par ailleurs, le microprocesseur est programmé de façon à pouvoir constater la survenue d'une défaillance cardiaque. Par exemple, le dispositif considère qu'un taux d'au moins 15 % d'extrasystoles ventriculaires identifiées signe une défaillance, de même que la détection d'une fibrillation ventriculaire. L'association de moyens de détection électronique et de pression du coeur est déjà décrite dans la littérature par exemple brevet français FR-A-1.237.702.

Lorsque le dispositif selon l'invention, qui est de préférence combiné à un défibrillateur automatique et à un dispositif anti-tachycardie tel que décrit dans le brevet US-A-4.052.991, détecte la survenue d'une défaillance cardiaque, il extrait de la mémoire 3 les valeurs des paramètres correspondant aux 9216 cycles qui ont précédé la survenue de la défaillance et les introduit dans la mémoire 4.

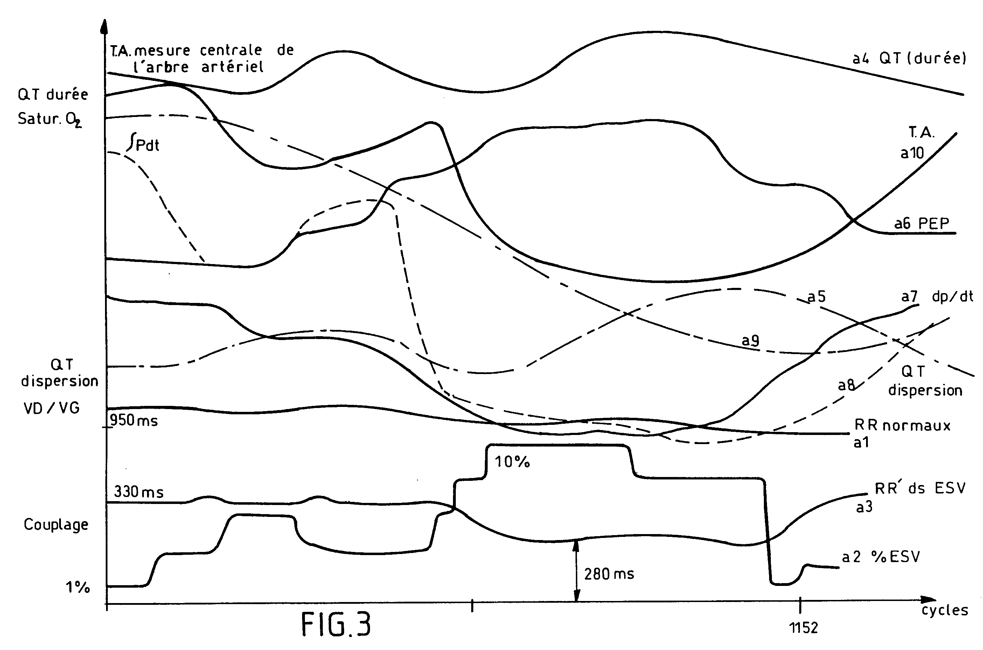

On a représenté, sur la figure 3, les courbes 1 à 10 des différents paramètres au cours d'une séquence de 1152 cycles consécutifs constitués par les cycles 1252 à 100 qui ont précédé la survenue de la défaillance.

Le dispositif selon l'invention compare en permanence les valeurs des différents paramètres précités pendant la séquence active en cours aux valeurs mémorisées et représentées sur les courbes(a1 ... a10) de la figure 3.

Pour la courbe a1 (RR), la comparaison s'effectue comme dans l'exemple 1.

Pour la courbe a2 (pourcentage d'extrasystoles), le dispositif estime qu'il y a ressemblance dans la comparaison dès lors qu'un nombre voisin d'extrasystoles a été compté pendant la séquence.

Il estime pour la courbe a3 (RR' des extrasystoles ventriculaires) que les courbes se ressemblent si plusieurs extrasystoles ayant un intervalle de couplage RR' inférieur à 350 ms ont été détectées.

En ce qui concerne la courbe a4 (intervalle QT), il estime que les courbes se ressemblent si elles présentent les mêmes amplitudes et vitesses d'accroissement.

En ce qui concerne la courbe a5 (dispersion de l'intervalle QT), il estime que les courbes se ressemblent si les variations de la dispersion, dans les deux courbes, sont supérieures à 50 ms et le rapport dispersion QT/durée minimale QT varie significativement.

Des critères de comparaison sont éventuellement retenus pour les courbes a6 à a10.

Le microprocesseur est programmé de la façon suivante :
- la comparaison positive des courbes a1 ne provoque l'intervention du dispositif que si la comparaison de l'une au moins des a2, a3, a6, a7 est positive.

Par contre une coïncidence des courbes a2 et/ou a3 entraînera la mise en oeuvre des moyens d'intervention du dispositif sans exiger que l'une au moins des autres courbes coïncide avec la courbe correspondante de la séquence active.

Dans l'exemple qui vient d'être décrit, la séquence active est comparée à la séquence représentée sur la figure 3 qui a été mémorisée et qui correspond aux cycles 1252 à 100 avant la défaillance qui a été enregistrée.

En fait, on peut, en variante, comparer la séquence active à un grand nombre de séquences mémorisées antérieures et on pourrait, par exemple, comparer la séquence active a l'une quelconque des séquences formées par 1152 cycles consécutifs dont le dernier est pris d'une façon arbitraire dans les cycles mémorisés dans la mémoire 4.

En variante, encore, le dispositif peut être agencé par programmation du microprocesseur de façon à retenir comme séquence mémorisée de comparaison, l'une des séquences de 1152 cycles consécutifs survenues avant la défaillance enregistrée et présentant une allure particulièrement caractéristique, par exemple un nombre élevé d'extrasystoles ventriculaires, un allongement du QT et un décalage important des intervalles QT sur deux dérivations différentes donnant une dispersion QT anormale.

En ce qui concerne les extrasystoles ventriculaires, le dispositif est, comme cela a été dit, de préférence combiné avec les moyens de réduction de tachycardie décrits dans la demande de brevet US précitée. Ce procédé détecte la survenue d'un certain nombre d'extrasystoles ou une tachycardie naissante et provoque automatiquement une stimulation isolée, ou de préférence une séquence de stimulations électriques, à une fréquence supérieure à celle des extrasystoles qui viennent d'être détectées. A la fin de la stimulation, le dispositif selon l'invention détecte si les extrasystoles persistent ou non. Si les extrasystoles ne persistent pas, le dispositif peut être agencé pour ne pas entraîner la mise en oeuvre des moyens d'intervention.

Si au contraire, les extrasystoles persistent, les moyens d'alerte et/ou d'intervention sont alors automatiquement mis en oeuvre.

## Revendications

1. Dispositif de prévention des défaillances cardiaques comportant des moyens de détection de paramètres cardiaques (6, 7, 8, 9) et des moyens d'intervention cardiaque (10, 11, 12) et/ou d'alerte, caractérisé en ce qu'outre des moyens pour la détection de la survenue de troubles cardiaques graves notamment arythmies ventriculaires et insuffisances mécaniques, il comporte des moyens de mémorisation (3) sensibles auxdits moyens de détection et susceptibles de garder au moins temporairement en mémoire la suite des valeurs des paramètres correspondant à une séquence active de cycles cardiaques et des moyens de comparaison (1), pour comparer ladite suite de valeurs de paramètres de ladite séquence active à des informations préalablement mémorisées de valeurs de paramètres, et, en cas de comparaison positive, pour déclencher la mise en oeuvre préventive desdits moyens d'intervention et/ou d'alerte.

2. Dispositif selon la revendication 1, caractérisé en ce qu'il comporte des moyens autoprogrammables permettant d'adapter le dispositif à l'évolution de l'état cardiovasculaire naturelle ou aux évolutions d'une part de prothèses placées au niveau du coeur et d'autre part des greffes tissulaires autologues ou hétérologues à cellules génétiquement modifiées ou non, d'une manière autonome par rapport à la programmation préétablie.

3. Dispositif selon l'une des revendications 1 et 2, caractérisé en ce qu'il est agencé pour conserver en mémoire une séquence active comprise entre 6 ou 8 cycles et quelques centaines ou milliers ou millions de cycles.

4. Dispositif selon l'une des revendications 1 à 3, caractérisé en ce qu'il est agencé pour conserver dans une mémoire (4) les valeurs de paramètres d'au moins une séquence ancienne qui a été suivie d'un incident cardiaque.

5. Dispositif selon les revendications 2 et 4, caractérisé en ce que le dispositif est agencé pour conserver automatiquement en mémoire ladite séquence ancienne au moment où il a détecté la défaillance.

6. Dispositif selon l'une quelconque des revendications 1 à 5, caractérisé en ce que les moyens de comparaison sont agencés pour constater une similitude entre la suite des valeurs de paramètres de la séquence active et de ladite séquence enregistrée ayant précédé une défaillance pour actionner lesdits moyens d'intervention en cas de constatation de similitude.

7. Dispositif selon l'une quelconque des revendication 1 à 6, caractérisé en ce qu'il est agencé pour conserver dans une mémoire (4) des valeurs de paramètres considérées comme normales chez le patient connecté au dispositif, les moyens de comparaison étant agencés pour comparer la suite des valeurs de paramètres de la séquence active auxdites informations et procéder au déclenchement des moyens d'intervention et/ou d'alerte en cas de différence significative.

8. Dispositif selon l'une quelconque des revendications 1 à 7, caractérisé en ce qu'il comporte des électrodes pour détecter des paramètres de type électrique, notamment choisis parmi les suivants : période et féquence cardiaques (RR), amplitude, morphologie et forme de l'électrocardiogramme, vectocardiogramme, complexe QRS, intervalle de l'onde T (QT), forme et/ou propagation de l'onde T, gradient de l'onde T, vectocardiogramme de l'onde T, détection de potentiel QRS retardé, impédances, nombre ou pourcentage ou morphologie des extrasystoles ventriculaires, intervalles de couplage RR' des extrasystoles ventriculaires, intervalle des complexes QRS et/ou des ondes T.

9. Dispositif selon l'une quelconque des revendications 1 à 6, caractérisé en ce qu'il comporte des capteurs susceptibles de détecter des paramètres de type chimique, et notamment saturation en oxygène ou CO_{2,} pH, acide lactique, concentration en ions notamment Ca⁺⁺, facteur de la coagulation, glutathion ou autres anti-oxydants, myosine, catécholamines, endotélime, radicaux libres.

10. Dispositif selon l'une quelconque des revendications 1 à 9, caractérisé en ce qu'il comporte des capteurs permettant de détecter des paramètres mécaniques, notamment PEP, dP/dt, diagramme pression, diagramme pression/ volume, volume du coeur, valeur de la pression d'éjection, intégrale de la pression, pression artérielle.

11. Dispositif selon l'une quelconque des revendications 1 à 10, caractérisé en ce qu'il comporte des moyens de détection intra-cardiaque.

12. Dispositif selon l'une des revendications 2 à 11, caractérisé en ce qu'il comporte des moyens de détection de l'effort et/ou des moyens procèdent à des tests d'exploration provoquant des variations de paramètres.

13. Dispositif selon l'une des revendications 2 à 12, caractérisé en ce qu'il comporte des moyens de détection de paramètres tels que les facteurs coagulants, anticoagulants, fibrinogéniques ou fibrinolytiques.

14. Dispositif selon l'une des revendications 1 à 13, caractérisé par des moyens de détection vectographiques du coeur.

15. Dispositif selon l'une quelconque des revendications 1 à 14, caractérisé par le fait qu'il comporte des moyens de télécommande actionnables de l'extérieur et permettant, à la demande, l'autoadministration par le porteur de médicaments appropriés, notamment trinitrine et/ou inhibiteur de la trombine et/ou fibrinolytique.

## Claims

1. Device for preventing heart failure, comprising means for detecting cardiac parameters (6, 7, 8, 9) and cardiac intervention and/or warning means (10, 11, 12), characterised in that, in addition to means for detecting the onset of serious heart disorders, especially ventricular arrhythmias and mechanical insufficiencies, it comprises memorising means (3) sensitive to said detecting means and capable of at least temporarily memorising the series of values of the parameters corresponding to an active sequence of cardiac cycles and comparison means (1) for comparing said sequence of values of parameters in said active sequence with previously memorised data relating to values of parameters, and, in the event of a positive comparison, for actuating the preventive implementation of said intervention and/or warning means.

2. Device according to claim 1, characterised in that it comprises auto-programmable means for autonomously adapting the device to the development of the natural cardiovascular state or to developments in, on the one hand, prostheses placed in the heart and, on the other hand, autologous or heterologous tissue grafts, which may or may not be genetically modified, relative to the pre-set programming.

3. Device according to one of claims 1 and 2, characterised in that it is arranged so as to store in its memory an active sequence of between 6 or 8 cycles and several hundreds or thousands or millions of cycles.

4. Device according to one of claims 1 to 3, characterised in that it is arranged so as to retain in a memory (4) the values of parameters of at least one old sequence which was followed by a cardiac incident.

5. Device according to claims 2 and 4, characterised in that the device is arranged so as to retain said old sequence in its memory automatically at the moment it detects a failure.

6. Device according to any one of claims 1 to 5, characterised in that the comparison means are arranged so as to detect any similarity between the series of values of parameters of the active sequence and said recorded sequence which preceded a failure, so as to actuate the intervention means when any similarity is detected.

7. Device according to any one of claims 1 to 6, characterised in that it is arranged so as to store, in a memory (4), the values of parameters which are regarded as normal in the patient connected to the device, the comparison means being adapted to compare the series of values of parameters of the active sequence with said data and proceed to actuate the intervention and/or warning means in the event of any significant variation.

8. Device according to any one of claims 1 to 7, characterised in that it comprises electrodes for detecting parameters of an electrical nature, particularly selected from the following: heart period and rate (RR), amplitude, morphology and shape of the electrocardiogram, vectocardiogram, QRS complex, T-wave interval (QT), form and/or propagation of the T-wave, gradient of the T-wave, vectocardiogram of the T-wave, detection of delayed QRS potential, impedances, number or percentage or morphology of ventricular extrasystoles, RR' coupling intervals of ventricular extrasystoles, interval of QRS complexes and/or T-waves.

9. Device according to any one of claims 1 to 6, characterised in that it comprises sensors capable of detecting chemical parameters, especially the oxygen or CO₂ saturation, pH, lactic acid concentration of ions, particularly Ca⁺⁺, coagulation factor, glutathion or other antioxidants, myosin, catecholamines, endotelime and free radicals.

10. Device according to any one of claims 1 to 9, characterised in that it comprises sensors adapted to detect mechanical parameters, notably PEP, dP/dt, pressure diagram, pressure/volume diagram, heart volume, value of expulsion pressure, pressure integral and arterial pressure.

11. Device according to any one of claims 1 to 10, characterised in that it comprises intracardiac detection means.

12. Device according to one of claims 2 to 11, characterised in that it comprises means for detecting stress and/or means for carrying out exploratory tests causing variations in parameters.

13. Device according to one of claims 2 to 12, characterised in that it comprises means for detecting parameters such as coagulant, anticoagulant, fibrinogenic or fibrinolytic factors.

14. Device according to one of claims 1 to 13, characterised by vectographic detecting means for the heart.

15. Device according to any one of claims 1 to 14, characterised in that it comprises remote-control means operable from outside which enable the user to administer appropriate drugs to himself, as required, especially trinitrin and/or thrombin inhibitors and/or fibrinolytic agents.

## Patentansprüche

1. Vorrichtung zur Verhinderung von Herzfunktionsstörungen, mit Einrichtungen (6,7,8,9) zur Ermittlung von Herzfunktions-Parametern und Einrichtungen, (10,11,12) zur Herzfunktions-Intervention und/oder zur Alarmgebung, dadurch **gekennzeichnet**, daß außer Einrichtungen zum Ermitteln des plötzlichen Auftretens von schweren Herzfunktionsstörungen, insbesondere ventrikulären Rhythmusstörungen und mechanischen Insuffizienzen die Vorrichtung Einrichtungen (3) zur Speicherung, die auf die Ermittlungseinrichtungen ansprechen und imstande sind, wenigstens zeitweilig im Speicher die Folge der Werte der Parameter festzuhalten, die einem aktiven Zyklus der Herzfunktion entsprechen, sowie Vergleichseinrichtungen (1) zum vergleichen der Folge der Werte der Parameter der aktiven Sequenz mit zuvor gespeicherten Informationen über die Werte der Parameter und im Falle eines positiven Vergleichsergebnisses zur vorsorglichen Auslösung der Einrichtungen zur Intervention und/oder zur Alarmgebung umfaßt.

2. Vorrichtung nach Anspruch 1, dadurch **gekennzeichnet**, daß die Vorrichtung selbst-programmierbare Einrichtungen umfaßt, die es gestatten, die Vorrichtung anzupassen an die Entwicklung des kardiovaskulären natürlichen Zustands oder an Entwicklungen einerseits von in Höhe des Herzens angeordneten Prothesen und andererseits autologen oder heterologen Gewebetransplantaten mit genetisch modifizierten oder nicht modifizierten Zellen unabhängig von der zuvor vorgesehenen Programmierung.

3. Vorrichtung nach einem der Ansprüche 1 und 2, dadurch **gekennzeichnet**, daß sie ausgebildet ist zur Aufbewahrung einer aktiven Sequenz von zwischen 6 und 8 Zyklen und einigen Hundert oder Tausend oder Millionen Zyklen im Speicher.

4. Vorrichtung nach einem der Ansprüche 1 bis 3, dadurch **gekennzeichnet**, daß sie ausgebildet ist zur Aufbewahrung der Werte der Parameter wenigstens einer früheren Sequenz, der eine Herzfunktionsstörung gefolgt ist, im Speicher (4).

5. Vorrichtung nach Anspruch 2 und 4, dadurch **gekennzeichnet**, daß die Vorrichtung ausgebildet ist zur automatischen Aufbewahrung der früheren Frequenz im Augenblick, in dem die Störung ermittelt wird, im Speicher.

6. Vorrichtung nach einem der Ansprüche 1 bis 5, dadurch **gekennzeichnet**, daß die Einrichtungen zum Vergleichen ausgebildet sind zur Feststellung einer Ähnlichkeit zwischen der Folge der Werte der Parameter der aktiven Sequenz und der registrierten Frequenz, die einer Störung vorausgegangen ist, zur Auslösung der Interventionseinrichtungen im Falle der Feststellung der Ähnlichkeit.

7. Vorrichtung nach einem der Ansprüche 1 bis 6, dadurch **gekennzeichnet**, daß sie ausgebildet ist zur Aufbewahrung der Werte der Parameter im Speicher (4), die bei dem an die Vorrichtung angeschlossenen Patienten als normal angesehen worden sind, wobei die Vergleichseinrichtungen ausgebildet sind zum Vergleichen der Folge der Werte der Parameter der aktiven Sequenz mit den erwähnten Informationen und zum Fortschreiten zur Auslösung der Einrichtungen zur Intervention und/oder zur Alarmgebung im Falle einer signifikanten Differenz.

8. Vorrichtung nach einem der Ansprüche 1 bis 7, dadurch **gekennzeichnet**, daß sie elektrische Elektroden zum Abtasten der Parameter, insbesondere von aus den folgenden ausgewählten Parametern umfaßt: Herzperiode und -frequenz (RR), Amplitude, Morphologie und Form des Elektrokardiogramms, Vektokardiogramms, des komplexen QRS, Wellenintervall T (QT), Form und Fortsetzung der Welle (T), Gradient der Welle (T), Vektokardiogramm der Welle (T), Erfassung eines potentiell verspäteten QRS, Impedanzen, Anzahl und Prozentsatz oder Morphologie der Extrasystolen des Herzens, Intervalle der Folge (RR') der Extrasystolen des Herzens, Intervalle des komplexen QRS und/oder der Wellen (T).

9. Vorrichtung nach einem der Ansprüche 1 bis 6, dadurch **gekennzeichnet**, daß sie Sensoren umfaßt, die in der Lage sind, chemische Parameter zu erfassen, insbesondere die Sättigung mit Sauerstoff oder CO₂, PH-Wert, Milchsäure, Ionenkonzentration, insbesondere Ca⁺⁺, Koagulationsfaktor, Glutation oder andere Antioxidantien, Myosine, Katecholamine, Endotelime, freie Radikale.

10. Vorrichtung nach einem der Ansprüche 1 bis 9, dadurch **gekennzeichnet**, daß sie Sensoren umfaßt, die die Abtastung von mechanischen Parametern gestattet, insbesondere PEP, dP/dt, Druckdiagramm, Druck-/Volumen-Diagramm, Herzvolumen, Druckwert des Ausstoßes, Integral des Druckes, Arteriendruck.

11. Vorrichtung nach einem der Ansprüche 1 bis 10, dadurch **gekennzeichnet**, daß sie eine Einrichtung zur intrakardialen Abtastung umfaßt.

12. Vorrichtung nach einem der Ansprüche 2 bis 11, dadurch **gekennzeichnet**, daß sie Einrichtungen zur Erfassung der Stärke und/oder Einrichtungen umfaßt, die Untersuchungstests durchführen, die Änderungen der Parameter provozieren.

13. Vorrichtung nach einem der Ansprüche 2 bis 12, dadurch **gekennzeichnet**, daß sie Einrichtungen zur Abtastung von Parametern, wie Koagulations- oder Antikoagulationsfaktoren und fibrinogenen oder fibrinolytischen Faktoren umfaßt.

14. Vorrichtung nach einem der Ansprüche 1 bis 13, **gekennzeichnet** durch Einrichtungen zur vektographischen Überwachung des Herzens.

15. Vorrichtung nach einem der Ansprüche 1 bis 14, dadurch **gekennzeichnet**, daß sie von außen betätigbare Fernsteuerungseinrichtungen umfaßt, die es gestatten, bei Bedarf die Verabreichung von geeigneten Medikamenten durch den Träger zu steuern, insbesondere von Trinitrin und/oder von Thrombose- und/oder fibrinolytischen Hemmern.
